# EUROPEAN PATENT APPLICATION

(11) **EP 3 199 114 A1**
(43) Date of publication of application: **02.08.2017**
(21) Application number: 17157796.8
(22) Date of filing: 20.12.2013
(51) Int. Cl.: A61B 17/72, A61B 17/84, A61B 17/86

(54) **PRECUTANEOUS FLARING HAMMERTOE FIXATION IMPLANT**

(30) Priority: 27.12.2012 US 201261746298 P; 03.04.2013 US 201313856181
(62) Divisional of application: 13199174.7
(71) Applicant: Wright Medical Technology, Inc., Memphis, TN 38117 (US)
(72) Inventor: McCORMICK, Daniel, Germantown, Tennessee 38138 (US)
(74) Representative: Gerbino, Angelo

(57) **Abstract**

A method and device to correct hammertoes. The device includes a bone implant having a screw with a threaded end or a pin and a sleeve adaptable to accept the screw or mate with the pin. The sleeve includes an external surface having threads on a distal end of the sleeve and an expanding feature along a proximate portion of the sleeve. When the implant is implanted into a joint through a percutaneous incision and the screw is rotated about its longitudinal axis when mated with the threaded portion of the sleeve, the expanding feature may be expanded compress the joint using the cancellous threads on a distal bone in the joint and the expanding feature on a proximate bone in the joint. In embodiments having pins, the pin is axially driven when locked in the sleeve by the locking mechanism thereby radially expanding the expanding feature to compress a joint using the cancellous threads on a distal bone in the joint and the expanding feature on a proximate bone in the joint.

## Description

### CROSS REFERENCES

The present application is co-pending with and claims the priority benefit of the provisional application entitled, "Percutaneous Flaring Hammertoe Fixation Implant and Instrument," Application Serial No. 61/746,298, filed on December 27, 2012.

### FIELD OF DISCLOSURE

The disclosed device generally relate to hammertoe correction implants and devices.

### BACKGROUND

A hammertoe or contracted toe is a deformity of the proximal inter-phalangeal joint of the second, third, or fourth toe causing it to be permanently bent and giving it a semblance of a hammer. Initially, hammertoes are flexible and may be corrected with simple measures but, if left untreated, hammertoes may require surgical intervention for correction. Persons with hammertoe may also have corns or calluses on the top of the middle joint of the toe or on the tip of the toe and may feel pain in their toes or feet while having difficulty finding comfortable shoes.

Various treatment strategies are available for correcting hammertoes. Conventionally, the first line of treatment for hammertoes includes employing new shoes having soft and spacious toe boxes. Additionally, toe exercises may be prescribed to stretch and strengthen respective muscles, e.g., gently stretching one's toes manually, using the toes to pick up things off the floor, etc. Another line of treatment may include employing straps, cushions or non-medicated corn pads to relieve symptoms. An addition method of treatment may include correction by surgery if other non-invasive treatment options fail. Conventional surgery usually involves inserting screws, wires or other similar implants in toes to straighten them. Traditional surgical methods generally include the use of Kirschner wires (K-wires). Due to various disadvantages of using K-wires, however, compression screws are being employed as a better implant alternative as K-wires require pings protruding through the end of respective toes due to their temporary nature. As a result, K-wires often lead to pin tract infections, loss of fixation, and other conditions. Additional disadvantages of K-wires include migration and breakage of the K-wires thus resulting in multiple surgeries.

Screw implants, however, may provide a more permanent solution as such implants do not need removal and thus have no protruding ends. Further, with the use of screw implants, a patient may wear normal footwear shortly after the respective surgery. Conventional screw implants possess a completely threaded body and do not provide a flexibility to the respective toe in its movement, i.e., conventional implants provide a pistoning effect. Furthermore, conventional screw implants are made for strong bones and are unsuitable for treatment of patients having weak bones which is a predominant reason why K-wire surgical implants are still employed despite their several disadvantages. Accordingly, there remains a need for developing hammertoe implants and devices which allow percutaneous implantation and can be expanded within the bone enabling faster operating and healing time.

### BRIEF DESCRIPTION OF THE DRAWINGS

Other objects, features, and advantages of the present subject matter will be apparent from the following description when read with reference to the accompanying drawings. In the drawings, wherein like reference numerals denote corresponding parts throughout the several views.
Figures 1A-1D illustrate views of an exemplary implant according to some embodiments of the present subject matter.
Figure 2 illustrates an insertion method of an exemplary implant according to an embodiment of the present subject matter.
Figures 3A-3C illustrate views of another exemplary implant according to embodiments of the present subject matter.
Figures 4A-4B illustrate views of a further exemplary implant according to embodiments of the present subject matter.
Figure 5 illustrates an insertion method of an exemplary implant according to another embodiment of the present subject matter.
Figures 6A-6D illustrate views of an exemplary implant according to some embodiments of the present subject matter.

### DETAILED DESCRIPTION

With reference to the figures, where like elements have been given like numerical designations to facilitate an understanding of the present subject matter, the various embodiments of a percutaneous flaring hammertoe fixation implant and instrument are described.

It should be noted that the figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures. The terms "implant" and "device" are used interchangeably in this disclosure and such use should not limit the scope of the claims appended herewith.

One embodiment of the present subject matter provides a driver and a hammertoe implant which interface to provide an efficient and effective delivery of the implant. The hammertoe implant or device includes an inner and outer body positioned coaxially to each other. The outer body possesses cancellous threads on the exterior surface from the distal end up to a flaring feature of the implant. The inner body may also include a mating feature commensurate with that of the inner body's design.

With reference to the figures herein, one aspect of embodiments of the present subject matter provide a percutaneous implantation for hammertoe corrective devices or implants. Exemplary implants can be driven into a predetermined location and anchored and expanded within the respective bone. This implantation may be accomplished with a single incision thus enabling a faster operating and healing time for the respective patient.

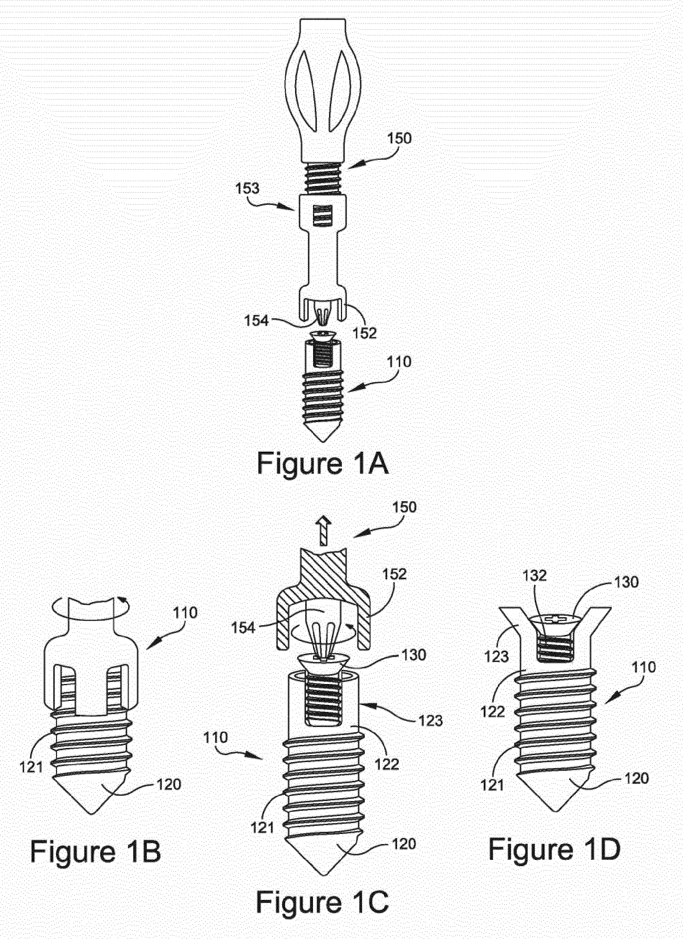

Figures 1A-1D illustrate views of an exemplary implant according to some embodiments of the present subject matter. Figure 1A is a front view of an exemplary hammertoe implant or device 110 with a driver 150 therefor, Figure 1B is a front view of the implant and outer portion of the driver of Figure 1A in an engaged position, Figure 1C is a front view of the implant and inner portion of the driver of Figure 1A in an engaged position, and Figure 1D is a front view of the implant in an expanded or flared position. With reference to Figures 1A-1D, an exemplary hammertoe implant or device 110 includes an outer body 120 and an inner body 130 or screw. The outer body 120 of the device 110, in some embodiments, comprises a sleeve 122 adaptable to accept the inner body 130. Generally, the outer diameter of the sleeve 122 substantially corresponds to the diameter of a pre-drilled hole in the receiving bone; however, the sleeve 122 may include flaring features 123 such as, but not limited to, a flaring collar or other mechanism, which allow expansion of an upper portion of the sleeve 122 when the inner body 130 is fully engaged with the outer body 120 to thereby anchor the device 110 in a bone and compress a respective joint. The sleeve 122 may also include, but is not limited to, cancellous threads 121 on an exterior surface thereof from the distal end of the device 110 up to the flaring feature 123 of the device 110. For example, while some embodiments of the present subject matter are described having cancellous threads, the claims appended herewith should not be so limited as exemplary sleeves may include any types of threading or threads. Within continued reference to Figures 1A-1D, these cancellous threads 121 may assist in the anchoring and/or location of the device or implant 110 in a respective bone. For example, an exemplary driver 150 may be employed to percutaneously implant the device 110 into a pre-drilled hole in a joint or bone of interest. The driver 150 includes an inner driver portion 154 and an outer driver portion 152. To implant an exemplary device 110, the outer driver portion 152 may interface with a mating feature such as, but not limited to, a flaring feature 123 of the device 110. Through rotation of the driver 150, the device 110 may be positioned in a predetermined location in a respective bone using, in part, the cancellous threads 121 along the exterior surface of the device 110. Once implanted to a desired depth, the outer driver portion 152 may be pulled back and/or locked in place using a lock-out feature 153 on the driver to expose the inner driver portion 154. The inner driver portion 154 may then be used to fully seat the inner body 130 within the outer body 120 as described below.

With continued reference to Figures 1A-1D, the inner body 130 generally comprises a screw 132 having a head and a threaded body extending in a perpendicular direction from the head. The sleeve 122 includes a first region adaptable to accept and mate to the head of the screw 132 when the screw 132 is inserted into the sleeve 122. The sleeve 122 may also include an interior threaded region which mates with the threaded body of the screw 132. Once the screw 132 is inserted into and mated with the sleeve 122, the screw 132 may be rotated about its axis (i.e., an axis of a pre-drilled hole in a receiving bone). As a function of the mating of the interior threaded region of the sleeve 122 with the threaded body of the screw 132, the screw 132 will pull the flaring features 123 of the sleeve 122 against the joint line (not shown) thus ensuring that the sleeve 122 acts as an anchoring mechanism in the respective bone and the screw 132 and sleeve 122 will act in conjunction to compress the respective joint.

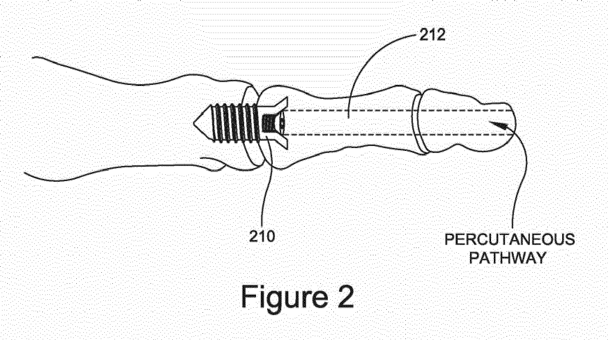

Figure 2 illustrates an insertion method of an exemplary implant according to an embodiment of the present subject matter. With reference to Figure 2, an exemplary implant 210 may be delivered to one or more of a patient's bones, in this case a metatarsal phalangeal joint, via a pre-drilled hole 212. Of course, the depicted metatarsal phalangeal joint should not limit the scope of the claims appended herewith as embodiments of the present subject matter may be employed in any number of bones and/or joints. The flaring features of the exemplary implant 210 as described above in relation to Figures 1A-1D will expand thus anchoring sections of the implant in the bone and compressing the respective joint. This may be achieved by mating threads of the inner and outer components or bodies the respective implant thus creating a secure construct.

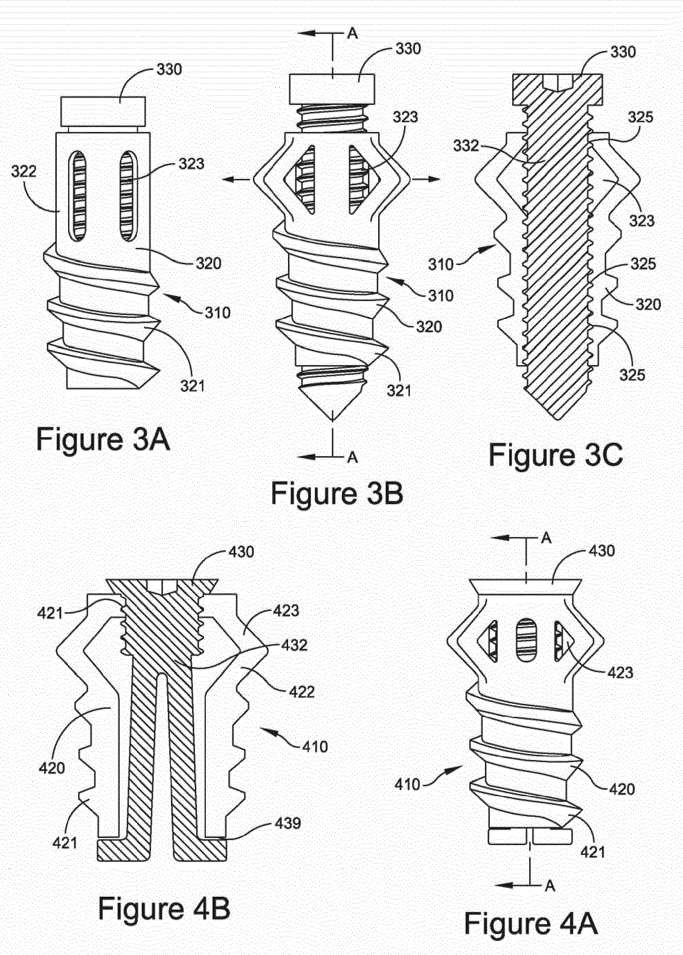

Figures 3A-3C illustrate views of another exemplary implant according to embodiments of the present subject matter. Figure 3A is a front view of an exemplary hammertoe implant or device 310, Figure 3B is a front view of the implant with a portion thereof in an engaged position, and Figure 3C is a cross section of the implant of Figure 3B along line A-A. With reference to Figures 3A-3C, an exemplary hammertoe implant or device 310 includes an outer body 320 and an inner body 330 or screw. The outer body 320 of the device 310, in some embodiments, comprises a sleeve 322 adaptable to accept the inner body 330. Generally, the outer diameter of the sleeve 322 substantially corresponds to the diameter of a pre-drilled hole in the receiving bone; however, the sleeve 322 may include flaring features 323 which allow expansion of an upper portion of the sleeve 322 when the inner body 330 is fully engaged with the outer body 320 to thereby anchor the device 310 in a bone and compress a respective joint. In the depicted embodiments, the flaring features 323 may be one or more slots extending down a portion of the length of the sleeve 322. Of course, any number and arrangement of slots may be included in embodiments of the present subject matter and the illustration of two slots in Figures 3A-3C should not limit the scope of the claims appended herewith. For example, the slots may extend in a spiral manner about portions of the sleeve and plural sets of slots may be arranged about the sleeve. The sets may be arranged about the length or width of the sleeve. Additionally, it is envisioned that a plurality of holes or other features may be arranged about the sleeve rather than the depicted slots to allow expansion of the sleeve when the inner body 330 is fully engaged with the outer body 320. The sleeve 322 may also include cancellous threads 321 on an exterior surface thereof from the distal end of the device 310 up to the flaring feature 323 of the device 310. These cancellous threads 321 may assist in the anchoring and/or location of the device or implant 310 in a respective bone. An exemplary driver as discussed with reference to Figures 1A-1D may be employed to percutaneously implant the device 310 into a pre-drilled hole in a joint or bone of interest.

With continued reference to Figures 3A-3C, the inner body 330 generally comprises a screw 332 having a head and a threaded body extending in a perpendicular direction from the head. The sleeve 322 includes a first region adaptable to accept and mate to the head of the screw 332 when the screw 332 is inserted into the sleeve 322. The sleeve 322 also includes an interior threaded region 325 which mates with the threaded body of the screw 332. Once the screw 332 is inserted into and mated with the sleeve 322, the screw 332 may be rotated about its axis (i.e., an axis of a pre-drilled hole in a receiving bone). As a function of the mating of the interior threaded region 325 of the sleeve 322 with the threaded body of the screw 332, the screw 332 will pull the flaring features 323 of the sleeve 322 against the joint line (not shown) thus ensuring that the sleeve 322 acts as an anchoring mechanism in the respective bone or joint and the screw 332 and sleeve 322 will act in conjunction to compress the respective joint.

Figures 4A-4B illustrate views of a further exemplary implant according to embodiments of the present subject matter. Figure 4A is a front view of an exemplary hammertoe implant or device 410, and Figure 4B is a cross section of the implant of Figure 4A along line A-A. With reference to Figures 4A-4B, an exemplary hammertoe implant or device 410 includes an outer body 420 and an inner body 430 or screw. The outer body 420 of the device 410, in some embodiments, comprises a sleeve 422 adaptable to accept the inner body 430. Generally, the outer diameter of the sleeve 422 substantially corresponds to the diameter of a pre-drilled hole in the receiving bone; however, the sleeve 422 may include flaring features 423 which allow expansion of an upper portion of the sleeve 422 when the inner body 430 is fully engaged with the outer body 420 to thereby anchor the device 410 in a bone and compress a respective joint. In the depicted embodiments, the flaring features 423 may be one or more slots extending down a portion of the length of the sleeve 422 as described with reference to Figures 3A-3C above. The sleeve 422 may also include cancellous threads 421 on an exterior surface thereof from the distal end of the device 410 up to the flaring feature 423 of the device 410. These cancellous threads 421 may assist in the anchoring and/or location of the device or implant 410 in a respective bone. An exemplary driver as discussed with reference to Figures 1A-1D may be employed to percutaneously implant the device 410 into a pre-drilled hole in a joint or bone of interest.

With continued reference to Figures 4A-4B, the inner body 430 generally comprises a screw 432 having a head and a partially threaded body extending in a perpendicular direction from the head. The screw 432 includes a distal locking mechanism 439 which acts to lock the screw 432 in the sleeve 422. The sleeve 422 includes a first region adaptable to accept and mate to the head of the screw 432 when the screw 432 is inserted into the sleeve 422. The sleeve 422 also includes an interior threaded region 421 which mates with the partially threaded body of the screw 432. Once the screw 432 is inserted into and mated with the sleeve 422, the screw 432 may be rotated about its axis (i.e., an axis of a pre-drilled hole in a receiving bone). As a function of the mating of the interior threaded region 421 of the sleeve 422 with the partially threaded body of the screw 432 and the locking of the screw 432 in the sleeve 422 by the distal locking mechanism 439, the screw 432 will pull the flaring features 423 of the sleeve 422 against the joint line (not shown) thus ensuring that the sleeve 422 acts as an anchoring mechanism in the respective bone and the screw 432 and sleeve 422 will act in conjunction to compress the respective joint.

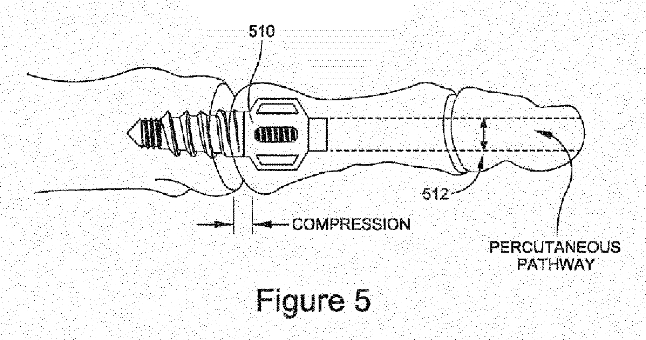

Figure 5 illustrates an insertion method of an exemplary implant according to another embodiment of the present subject matter. With reference to Figure 5, an exemplary implant 510 may be delivered to one or more of a patient's bones, in this case a metatarsal phalangeal joint, via a pre-drilled hole 512. Slotted features of the exemplary implant 510 as described above in relation to Figures 3A-3C will expand thus anchoring sections of the implant in the bone and compressing the respective joint line. This may be achieved by mating threads of the inner and outer components or bodies the respective implant thus creating a secure construct.

It is an aspect of some embodiments that once a respective implant has been positioned to the desired depth and location, the inner body thereof may be further advanced distally into the outer body where the head of the inner body interferes with the outer body wings or features. This may cause portions of the outer body to flare outwards. Such a design may enable the outer body's external threading to compress the distal end of the joint line while the flared wings compress the proximal side of the joint line thereby resulting in a construct which compresses a respective joint while fully fixated within the surgical site. Another aspect of embodiments of the present subject matter provide a driver having a coaxially positioned inner and outer portion. The driver outer portion interfaces with the outer body of the implant whereas the driver inner portion interfaces with the inner body of the implant. Both the inner and outer portions of the driver and implant may thus interface independently of each other and include respective mating features providing a strong torsional stability. Once an implant is driven to a desired location and depth, the driver outer portion may be drawn back axially and locked in place to expose the inner driver portion which is then used to drive the inner body of the implant. This results in an efficient method whereby an implant can be inserted and expanded leading to a fixated joint line with a single insertion. Such a method enables faster healing time and drastically faster operating time.

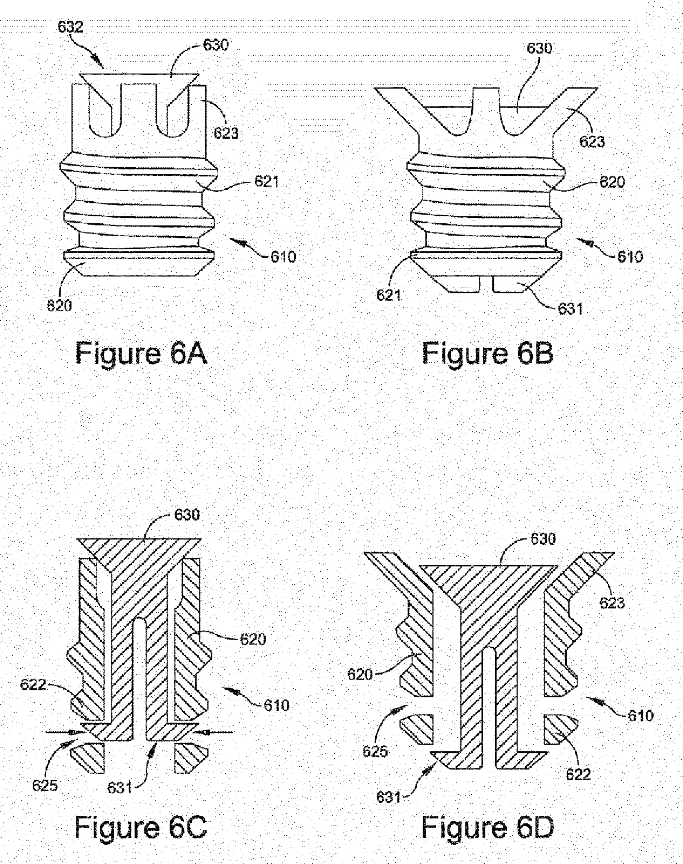

Figures 6A-6D illustrate views of an exemplary implant according to some embodiments of the present subject matter. Figure 6A is a front view of an exemplary hammertoe implant or device 610, Figure 6B is a front view of the implant of Figure 6A in an engaged position, Figure 6C is a cross section of the implant of Figure 6A, and Figure 6D is a cross section of the implant of Figure 6B. With reference to Figures 6A-6D, an exemplary hammertoe implant or device 610 includes an outer body 620 and an inner body 630. The outer body 620 of the device 610, in some embodiments, comprises a sleeve 622 adaptable to accept the inner body 630. Generally, the outer diameter of the sleeve 622 substantially corresponds to the diameter of a pre-drilled hole in the receiving bone; however, the sleeve 622 may include flaring features 623 which allow expansion of an upper portion of the sleeve 622 when the inner body 630 is fully engaged with the outer body 620 to thereby anchor the device 610 in a bone and compress a respective joint. The sleeve 622 may also include cancellous threads 621 on an exterior surface thereof from the distal end of the device 610 up to the flaring feature 623 of the device 610. These cancellous threads 621 may assist in the anchoring and/or location of the device or implant 610 in a respective bone.

With continued reference to Figures 6A-6D, the inner body 630 generally comprises a pin 632 having a head and a threadless body extending in a perpendicular direction from the head. The sleeve 622 includes a first region adaptable to accept and mate to the head of the pin 632 when the pin 632 is inserted into the sleeve 622. The sleeve 622 may also include a slotted region 625 which acts to mate or lock with locking tabs 631 on the pin 632. Once the pin 632 is inserted into and mated with the sleeve 622, the pin 632 may be pushed axially along its axis (i.e., an axis of a pre-drilled hole in a receiving bone). As a function of the mating of the pin 632 with the sleeve 622 using the locking tabs 631 and slotted region 625, the pin 632 will push the flaring features 623 of the sleeve 622 radially outward against the joint line (not shown) thus ensuring that the sleeve 622 acts as an anchoring mechanism in the respective bone and the pin 632 and sleeve 622 will act in conjunction to compress the respective joint. An exemplary driver may be employed to percutaneously implant the device 610 into a pre-drilled hole in a joint or bone of interest. Similar to the driver described above, the driver may include an outer driver portion but an inner plunger portion. To implant an exemplary device 610, the outer driver portion may interface with a mating feature or the flaring feature 623 of the device 610. Through rotation of the driver, the device 610 may be positioned in a predetermined location in a respective bone using, in part, the cancellous threads 621 along the exterior surface of the device 610. Once implanted to a desired depth, the outer driver portion may be pulled back and/or locked in place using a lock-out feature on the driver to expose the inner plunger portion. The inner plunger portion may then be used to fully mate the inner body 630 within the outer body 620 as described above.

It is also an aspect of some embodiments where an implant includes an outer body and an inner body, the inner body having a tapered head and a shaft with a split barbed locking feature. The outer body of the implant may be externally threaded and may include axial slotted features and a distal perpendicular slot feature. Another aspect of embodiments of the present subject matter provide a driver having an inner and outer portion positioned coaxially. The driver outer portion interfaces with the outer body of the implant whereas the driver inner portion interfaces with the inner body of the implant. The inner body of the implant may be rotationally driven into the desired position and depth such that the axial slotted features reside on the proximal side of the joint line and the threaded body resides on the opposing side. The lockout feature is disengaged and a plunger on the driver activated which forces the inner body of the implant to displace distally into the outer body thereby causing the slotted features of the outer body to radially expand outward due to the interference with the tapered head of the inner body. This may also disengage the implant from the driver whereby compression may be created between the expanded wings and the threads.

Although reference has been made to a patient's metatarsal phalangeal joint, one skilled in the art will understand that embodiments of the present subject matter may be implemented for other respective bones including, but not limited to other phalanges/digits and phalangeal/digital joints.

It may be emphasized that the above-described embodiments, particularly any "preferred" embodiments, are merely possible examples of implementations and merely set forth for a clear understanding of the principles of the disclosure. Many variations and modifications may be made to the above-described embodiments of the disclosure without departing substantially from the spirit and principles of the disclosure. All such modifications and variations are intended to be included herein within the scope of this disclosure and the present disclosure and protected by the following claims.

While this specification contains many specifics, these should not be construed as limitations on the scope of the claimed subject matter, but rather as descriptions of features that may be specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

As shown by the various configurations and embodiments illustrated in Figures 1-6D, a percutaneous flaring hammertoe fixation implant and instrument have been described.

While preferred embodiments of the present subject matter have been described, it is to be understood that the embodiments described are illustrative only and that the scope of the invention is to be defined solely by the appended claims when accorded a full range of equivalence, many variations and modifications naturally occurring to those of skill in the art from a perusal hereof.

## Claims

1. A bone implant comprising:
a pin having a distal locking mechanism and a head,; and
a sleeve accepting the pin, the sleeve comprising:
an external surface having threads on a distal end of the sleeve,
one or more distal locking slots to accept the distal locking mechanism of the pin, and
an expanding feature along a proximate portion of the sleeve and capable of being expanded by the head,
wherein the implant is implanted into a joint through a percutaneous incision, and
wherein after the pin is locked in the sleeve by the locking mechanism, the expanding feature is expanded and a joint is compressed by using the head .

2. The bone implant of Claim 1 wherein the expanding feature is selected from the group consisting of one or more slots extending down a portion of the sleeve, a plurality of holes arranged in a pattern about a portion of the sleeve, one or more slots arranged in a spiral down a portion of the sleeve, a flaring collar, and combinations thereof.

3. The bone implant of Claim 1 wherein the bone is a phalange selected from the group consisting of proximal phalange, intermediate phalange, distal phalange, and combinations thereof.
